(19) Europäisches Patentamt
European Patent Office
Office européen des brevets

(11) Publication number: **0 498 830 B1**

# (12) EUROPEAN PATENT SPECIFICATION

(45) Date of publication of patent specification: **14.12.94**

(51) Int. Cl.5: **C07C 237/22**, A61K 31/195

(21) Application number: **90916273.7**

(22) Date of filing: **25.10.90**

(86) International application number:
**PCT/EP90/01808**

(87) International publication number:
**WO 91/06529 (16.05.91 91/11)**

## (54) GLUTAMIC AND ASPARTIC ACID DERIVATIVES WITH ANTIGASTRIN ACTIVITY AND A METHOD FOR THEIR PREPARATION.

(30) Priority: **02.11.89 IT 6793789**

(43) Date of publication of application:
**19.08.92 Bulletin 92/34**

(45) Publication of the grant of the patent:
**14.12.94 Bulletin 94/50**

(84) Designated Contracting States:
**AT BE CH DE ES FR GB IT LI NL**

(56) References cited:

**EUROPEAN JOURNAL OF MEDICINAL CHEMISTRY - CHIMICA THERAPUETICA, vol. 21, no. 1, 1968, Chatenay-Malabry, FR; F. MAKOVEC et al, "New glutamic and aspartic derivatives with potent CCK-anatgonistic activity", pages 9-20**

(73) Proprietor: **ROTTA RESEARCH LABORATORIUM S.P.A.**
**Via Valosa di Sopra, 7**
**I-20052 Monza (Milano) (IT)**

(72) Inventor: **MAKOVEC, Francesco**
**Via Boito, 72**
**I-20052 Monza (IT)**
Inventor: **ROVATI, Claudio, Lucio**
**Via Cagni, 8**
**I-20052 Monza (IT)**
Inventor: **ROVATI, Angelo, Luigi**
**Via Valosa di Sopra, 28**
**I-20052 Monza (IT)**

(74) Representative: **Rambelli, Paolo et al**
**c/o JACOBACCI & PERANI S.p.A.**
**Corso Regio Parco, 27**
**I-10152 Torino (IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

The subject of the present invention is glutamic and aspartic acid derivatives and their use for the preparation of medicaments for the therapeutic treatment of disorders induced by hypergastrinaemia. These derivatives are represented by the general formula indicated below:

```
         CO OH
         ¦
         (CH2)n
         ¦
(*)      CH-NH-CO-R1              (I)
         ¦
         CO-NH-R2
```

in which n is 1 or 2, $R_1$ is a phenyl group mono- or di-substituted with chloro, methyl or nitro groups such that hydrogen or the nitro group may be present in positions 2 and 6 of the phenyl group and hydrogen, chloro or a methyl group may be present in positions 3 and 5, and in which $R_2$ is a linear or branched alkyl chain or a cyclo-alkyl group containing from 5 to 9 carbon atoms, the stereochemistry at the chiral centre marked with an asterisk in formula (I) being DL or D, including their pharmaceutically acceptable salts.

In particular, the preferred compounds within the scope of the invention are those described specifically in tables A and D below and their pharmaceutically acceptable salts, the compounds most preferred being those in which n is 2, $R_1$ is selected from the group consisting of 3-chlorophenyl and 3,5-dichlorophenyl and $R_2$ is selected from the group consisting of 3,3-dimethylbutyl, 4,4-dimethylpentyl and 3,3-methylethylpentyl.

The compounds of the present invention display powerful antagonistic activities towards gastrin ("little gastrin" or G-17) and towards pentagastrin which is its biologically active terminal peptide sequence.

Gastrin is a polypeptide hormone secreted by the cells of the antral and duodenal mucosae, its secretion being induced, in particular, by a nervous mechanism through vagal excitation.

Once secreted, gastrin flows, via the bloodstream to the parietal cells situated mainly in the mucosa fundica and, by binding to the membrane receptors of the cells, activates them. This activation initiates the formation of HCl and its secretion into the gastric cavity.

Hyperactivity of this system results in the hypersecretion of gastric acid which may have consequences harmful to the organism.

The compounds of the present invention belong to a more general class of glutamic and aspartic acid derivatives which are generally described in U.S. Patent A-4 791 215 in the name of the Applicant. Amongst the various activities mentioned with reference to the class of compounds which is the subject of the above patent, those relating to their powerful activities in inhibiting cholecystokinin (CCK) in various experimental models were of particular value. For example, the compound designated C-7 (lorglumide) inhibited the contraction induced by CCK-8 (10ng/ml) in guinea-pig gall bladders in vitro by 50% at a concentration of 0.06 mcg/ml, which is approximately 15 times greater than that of the antagonist. The compounds described specifically in the above patent were, however, inactive or only slightly active as inhibitors of pentagastrin (see, for example Eur. J. Med.Chem. (1986)-21, pp. 9-20) even though gastrin is closely related to CCK since the C-terminal pentapeptide Gly-Trpt-Met-Asp-Phe$NH_2$ is common to the two polypeptides. This led to the assumption that they interacted mainly with the tripeptide sequence CCK-(26-28) without interfering with the receptor structure which is responsible for the interaction with the terminal pentapeptide sequence CCK-(29-33) which is common to the two hormones CCK and gastrin, which is thus the opposite of what takes place with the compounds of the subject invention, which have powerful antigastrin activities and little or no activity against CCK.

The present invention therefore arises from the following considerations: the anti-CCK activities of the compounds of the patent mentioned above are due to the simultaneous presence of an acid group which acts as a solubiliser and of 3 suitably sized and spaced lipophilic units, that is, two alkyl substituents (corresponding to two $R_2$ groups in formula I) and a suitably substituted phenyl ring (corresponding to the substituent $R_1$ in formula I).

In order to obtain compounds with high antigastrin activities, however, as in the case of the compounds of the invention, the presence of the acid group and of the phenyl ring ($R_1$) with suitable substituents (which do not, however, coincide with those of the other series, that is, with the anti-CCK compounds) are still necessary, whilst the presence of only one hydrophobic alkyl group of suitable length and steric bulk is essential at $R_2$.

A further subject of the present invention is the use of the derivatives of formula (I) in which n, $R_1$ and $R_2$ have the meanings defined above, and their pharmaceutically acceptable salts, for the preparation of a medicament for the therapeutic treatment of conditions induced by hypergastrinaemia.

As already mentioned, the compounds of the invention have powerful and specific antigastrin activities in various experimental models, both in vivo and in vitro. In fact, at little more than nanomolar concentrations, the most powerful of these inhibit the binding of pentagastrin tritiate to the gastric mucous glands of guinea pigs and to the cortical membranes of mice.

In vivo, they show high activities against the secretion of acid, blocking the secretory stimulus induced by pentagastrin in a dose-dependent manner. This activity is specific since the compounds which are the suject of the invention do not block secretions induced, for example, by carbachol or histamine.

The compounds can thus be used, to advantage, in the treatment and prevention of various disorders in man in which it is advantageous to block the stimulant activity of gastrin, for example, ulcers or gastroduodenitis resulting from excessive gastric secretion induced by a hypergastrinaemic condition, in Zollinger-Ellison syndrome, or in the treatment of several forms of tumour which are sustained by hypergastrinaemia.

On the basis of their powerful activities displayed as inhibitors of the binding of gastrin at the level of the CNS, the compounds in question could also display activity towards some kinds of mental disorders attributable to an imbalance in the physiological neuron levels of gastrin or other bioactive peptides related thereto.

The method of preparing the derivatives of the invention with chiral centres in the DL form is characterised by the following steps which may be represented thus:

a) reacting an internal anhydride of formula:

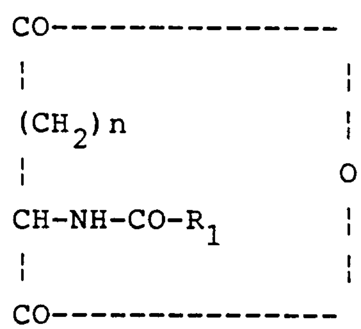

in which n and $R_1$ have the meanings given above, with a primary amine of formula $R_2$-$NH_2$, in which $R_2$ has the meaning given above, in a molar ratio of from 1 to 5, in a solvent such as water, ethyl acetate, dioxan, acetonitrile, tetrahydrofuran or a mixture thereof, at a temperature of between -20 °C and 30 °C and recovering the compounds of formula I from the reaction mass by fractional crystallisation or other conventional methods.

The following example is given in order better to illustrate the invention.

<u>Preparation of ( ± )-4-[(3-chlorobenzoyl)-amino]-5-(3, 3-dimethylbutylamino)-5-oxopentanoic acid (compound A-5 in Table Z)</u>

26.8g (0.1 moles) of 3-chlorobenzoyl glutamic anhydride were loaded into a reactor and suspended in 100 ml of water. The suspension was cooled to approximately 5 °C and 25.3g (0.25 moles) of 3,3-dimethylbutylamine were added dropwise over a period of approximately 15 minutes. The mixture was left to react for 3 hours at this temperature and then acidified with glacial acetic acid. It was filtered, washed with water until neutral and dried. The crude product obtained was crystallised from alcohol/$H_2O$ (3:2). 19.6 g were obtained. Yield 53%. M.P.: 194-195 °C. TLC (isoamyl alcohol-acetone-$H_2O$:5/2/1 - V/V): Rf. 0.78.

Table A below shows, by way of example, many examples of these compounds with some of their identifying characteristics, as well as the yields obtained.

TABLE A: derivatives (DL-series) having the formula:

```
CO OH
|
(CH2)n
|
CH-NH-CO-R1
|
CO-NH-R2
```

| COM-POUNDS | N | R1 | R2 | MELTING POINT | CRYSTALLISATION SOLVENT | Rf(*) | FORMULA | YIELD |
|---|---|---|---|---|---|---|---|---|
| A1 | 2 | 3-chlorophenyl | pentyl | 127-129 | Alcohol/$H_2O$ 1:1 | 0.78 | $C_{17}H_{23}ClN_2O_4$ | 57 |
| A2 | 2 | 3-chlorophenyl | 3-methylbutyl (isopentyl) | 159-160 | Alcohol/$H_2O$ 1:1 | 0.85 | $C_{17}H_{23}ClN_2O_4$ | 25 |
| A3 | 2 | 3-chlorophenyl | hexyl | 124-125 | Alcohol/$H_2O$ 1:1 | 0.75 | $C_{18}H_{25}ClN_2O_4$ | 43 |
| A4 | 2 | 3-chlorophenyl | 4-methylpentyl (isohexyl) | 137-138 | Alcohol/$H_2O$ 1:1 | 0.89 | $C_{18}H_{25}ClN_2O_4$ | 40 |
| A5 | 2 | 3-chlorophenyl | 3,3-dimethyl-butyl | 194-195 | Alcohol/$H_2O$ 3:2 | 0.78 | $C_{18}H_{25}ClN_2O_4$ | 53 |
| A6 | 2 | 3-chlorophenyl | 3-methylpentyl | 146-147 | Alcohol | 0.75 | $C_{18}H_{25}ClN_2O_4$ | 33 |
| A7 | 2 | 3-chlorophenyl | cyclohexyl | 200-202 | Alcohol | 0.82 | $C_{18}H_{23}ClN_2O_4$ | 74 |
| A8 | 2 | 3-chlorophenyl | heptyl | 122-124 | Alcohol/$H_2O$ 1:1 | 0.86 | $C_{19}H_{27}ClN_2O_4$ | 40 |
| A9 | 2 | 3-chlorophenyl | 4,4-dimethyl-pentyl | 162-163 | Alcohol/$H_2O$ 1:1 | 0.78 | $C_{19}H_{27}ClN_2O_4$ | 33 |
| A10 | 2 | 3-chlorophenyl | 3,3-dimethyl-pentyl | 190-191 | Alcohol/$H_2O$ 7:3 | 0.81 | $C_{19}H_{27}ClN_2O_4$ | 53 |
| A11 | 2 | 3-chlorophenyl | 3-ethylpentyl | 142-143 | Alcohol | 0.84 | $C_{19}H_{27}ClN_2O_4$ | 32 |

TABLE A - Continued

| COMPOUNDS | N | R1 | R2 | MELTING POINT | CRYSTALLISATION SOLVENT | Rf(*) | FORMULA | YIELD |
|---|---|---|---|---|---|---|---|---|
| A12 | 2 | 3-chlorophenyl | cycloheptyl | 200-202 | Methanol | 0.80 | $C_{19}H_{25}ClN_2O_4$ | 78 |
| A13 | 2 | 3-chlorophenyl | 3,3-methyl-ethylpentyl | 190-192 | Methanol | 0.89 | $C_{20}H_{29}ClN_2O_4$ | 31 |
| A14 | 2 | 3-chlorophenyl | 3,3-diethyl-pentyl | 177-179 | Alcohol | 0.90 | $C_{21}H_{31}ClN_2O_4$ | 47 |
| A15 | 2 | 3-chlorophenyl | 2-(dicyclo [2.2.1] heptylethyl | 185-186 | Alcohol | 0.82 | $C_{21}H_{27}ClN_2O_4$ | 36 |
| A16 | 2 | 3-chlorophenyl | 3,3-methylethyl-4-methylpentyl | 184-185 | Alcohol | 0.86 | $C_{21}H_{31}ClN_2O_4$ | 63 |
| A17 | 2 | 3-methylphenyl | pentyl | 111-114 | Alcohol/$H_2O$ 1:1 | 0.82 | $C_{18}H_{26}N_2O_4$ | 39 |
| A18 | 2 | 3-methylphenyl | hexyl | 107-111 | Alcohol/$H_2O$ 1:1 | 0.75 | $C_{19}H_{28}N_2O_4$ | 31 |
| A19 | 2 | 3-methylphenyl | cyclohexyl | 200-203 | Alcohol/$H_2O$ 3:1 | 0.69 | $C_{19}H_{26}N_2O_4$ | 40 |
| A20 | 2 | 3-methylphenyl | 3,3-dimethyl-pentyl | 178-181 | Alcohol/$H_2O$ 1:1 | 0.85 | $C_{20}H_{30}N_2O_4$ | 27 |
| A21 | 2 | 3,5-dichloro-phenyl | 3,3-dimethyl butyl | 230-232 | Alcohol | 0.83 | $C_{18}H_{24}Cl_2N_2O_4$ | 41 |
| A22 | 2 | 3,5-dimethyl-phenyl | 3,3-methyl-ethylpentyl | 198-201 | Alcohol/$H_2O$ 1:1 | 0.81 | $C_{22}H_{34}N_2O_4$ | 64 |
| A23 | 2 | 2-nitro-5-chlorophenyl | 3,3-methyl-ethylpentyl | 183-184 | Alcohol/$H_2O$ 1:1 | 0.85 | $C_{20}H_{28}ClN_3O_6$ | 28 |
| A24 | 1 | 3-chlorophenyl | 3,3-methyl-ethylpentyl | 164-165 | Alcohol/$H_2O$ 1:1 | 0.74 | $C_{19}H_{27}ClN_2O_4$ | 48 |
| A25 | 1 | 3-chlorophenyl | cycloheptyl | 181-183 | Alcohol/$H_2O$ 1:1 | 0.78 | $C_{18}H_{23}ClN_2O_4$ | 54 |
| A26 | 1 | 3-methylphenyl | 3,3-methyl-ethylpentyl | 147-148 | Alcohol/$H_2O$ 1:1 | 0.68 | $C_{20}H_{30}N_2O_4$ | 44 |

(*) eluent: isoamyl alcohol/acetone/water :5/2/1-V/V

The method for the preparation of the derivatives of the invention with chiral centres in the D form is characterised by the following steps, which may be represented thus:

a) reacting the gamma-benzyl ester of N-carbobenzoxy-D-glutamic acid with an amine of formula $R_2$-$NH_2$, in which $R_2$ has the meaning given above, by the mixed anhydride method, in an inert anhydrous solvent, at a temperature of between -15° and +15° to give compounds of formula (III); (see the scheme below)

b) debenzylating and decarbobenzoxylating the compound of formula (III) dissolved in an inert solvent by reacting it with hydrogen at ambient temperature and atmospheric pressure, in the presence of a catalytically effective quantity of a hydrogenation catalyst to obtain derivatives of formula (II) (see the scheme below);

c) reacting the derivatives of formula (II) under Schotten-Baumann conditions with an equivalent quantity of an acyl chloride of formula $R_1$-COCl, in which $R_1$ has the meaning given above, at a temperature of from 0 to 15°C and recovering the D-4-acylamino-5-alkylamino-5-oxopentanoic derivative of formula (I) from the reaction mass.

The sequence of steps of the method according to the invention is illustrated as a whole by the following scheme:

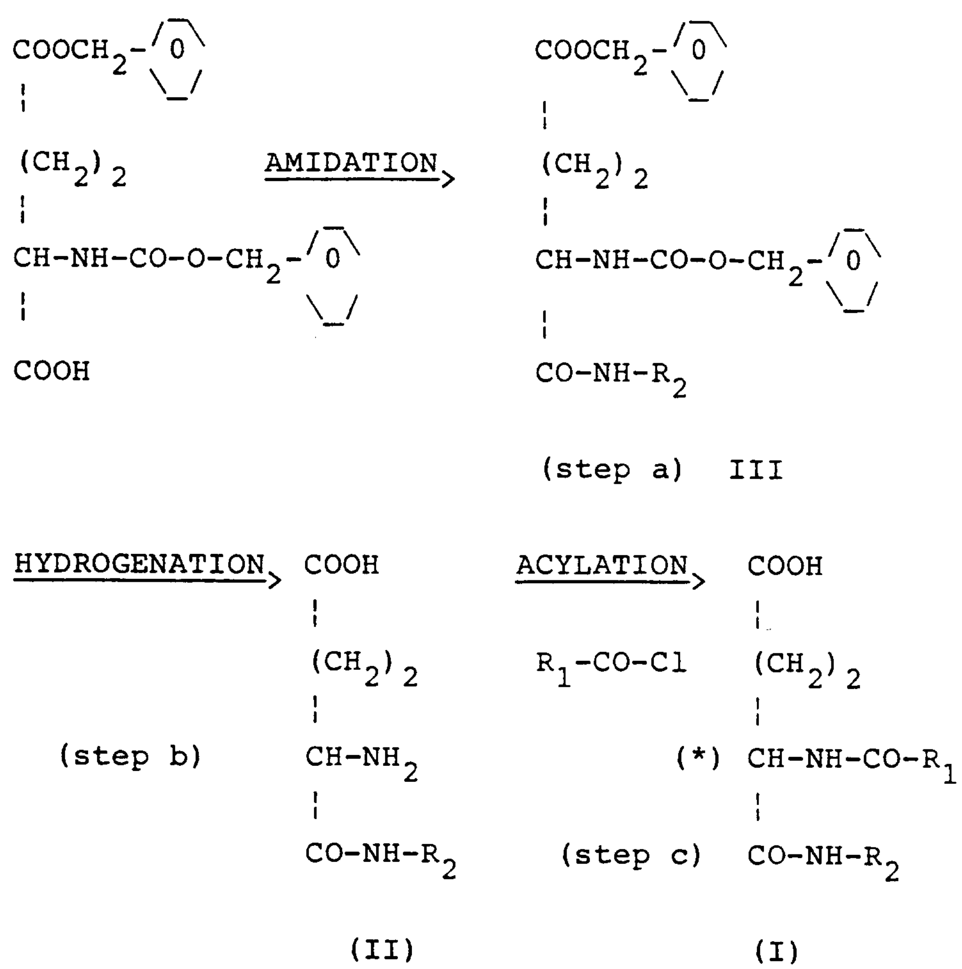


(*) chiral centre in the D form.

The amidation step (a) is carried out at a temperature preferably of between -15°C and -10°C for a period of from 1 to 24 hours, preferably for 6 hours, with the reagents in a stoichiometric ratio. The preferred solvent for the reaction is selected from chloroform, dioxan and tetrahydrofuran.

The hydrogenation step (b) is preferably carried out in the presence of a quantity of between 0.02 and 0.001 atoms of palladium per mole of compound (III), supported on carbon, in a methanolic solution, at ambient temperature and pressure, in a stream of hydrogen and for a period of from 1 to 12 hours, preferably for 3 hours. The acylation step (c) is preferably carried out at a temperature of approximately 5°C, over a period of from 1 to 24 hours, preferably for 12 hours.

The following examples are given in order better to illustrate the invention.

Example 2

Preparation of the benzyl ester of D-4-carbobenzoxyamino-5-(3,3-dimethylbutylamino)-5-oxopentanoic acid (compound $B_1$ of Table B).

37.1g (0.1 moles) of the gamma-benzyl ester of N-carbobenzoxy-D-glutamic acid was dissolved in 250 ml of anydrous tetrahydrofuran, the solution was cooled to -10 ° C and 10.1 g (0.1 moles) of triethyalmine were added under agitation: 10.8g (0.1 moles) of ethyl chlorocarbonate were then added, still at -10 ° C. The temperature was kept at -10 ° C for 20 minutes and 10.1 g (0.1 moles) of 3,3-dimethylbutylamine were then added. The mixture was left under agitation for a further 6 hours, the temperature rising to ambient temperature; the mixture was evaporated to dryness and the residue taken up with ethyl acetate.

This was washed with 2N HCl, sodium bicarbonate and finally water and then dried over anhydrous $Na_2SO_4$. By concentration to a small volume, an oily residue was obtained (mw 454.5) which crystallised spontaneously upon standing. The crude product obtained was crystallised from dioxan-water (1:1). TLC: Rf 0.78 [methylene chloride-alcohol 9/1 (V/V)]. Melting point: 104-106 ° C.

35.0 g were produced. Yield 77%.

All the compounds of formula III were synthesised by the same method (see the schemes above). Table B below gives some of the compounds thus obtained with some of their identifying characteristics.

TABLE B Derivatives having the formula:

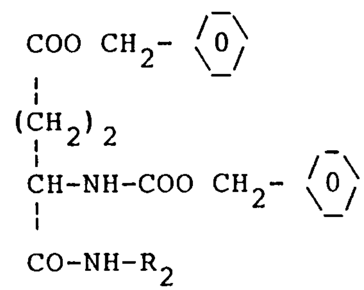

| COMPOUND | $R_2$ | MELTING POINT (°C) | Rf | FORMULA |
|---|---|---|---|---|
| $B_1$ | 3,3-dimethylbutyl | 104-106 | 0.78(1) | $C_{26}H_{34}N_2O_5$ |
| $B_2$ | 4,4-dimethylpentyl | waxy solid | 0.83(1) | $C_{27}H_{36}N_2O_5$ |
| $B_3$ | 3,3-methylethylpentyl | waxy solid | 0.88(1) | $C_{28}H_{38}N_2O_5$ |
| $B_4$ | Cycloheptyl | 151-153 | 0.49(2) | $C_{27}H_{34}N_2O_5$ |

(1) eluent: Methylene chloride/alcohol (9:1)

(2) eluent: Chloroform/ethyl acetate (9:1)

Example 3

Preparation of D-4-amino-5-(3,3-dimethylbutyl-5-oxopentanoic acid (Compound $C_1$ in Table C).

45.4g (0.1 moles) of the benzyl ester of D-4-carbobenzoxy-amino-5-(3,3-dimethylbutyl)-5-oxopentanoic acid (Compound $B_1$) were dissolved in 300 ml of methanol to which 1 g of 10% palladiated carbon had been added and hydrogenated at ambient temperature with a stream of hydrogen for 3 hours. The catalyst was filtered out and the methanol distilled under vacuum. An oily residue (mw 286.4) was obtained which crystallised upon standing and had a chromatographic purity of more than 95%. TLC: Rf 0.62 [n-butanol-acetic acid-$H_2O$ 5/2/2 (V/V)]. Melting point: 132-133 °C.

18.6 g were produced. Yield 81%.

All the compounds of formula II were synthesised by the same method (see scheme).

Table C below gives the compounds thus obtained with some of their identifying characteristics.

TABLE C Derivatives having the formula:

COOH
|
$(CH_2)_2$
|
$CH-NH_2$
|
$CO-NH-R_2$

| COMPOUND | $R_2$ | MELTING POINT (°C) | Rf (*) | FORMULA |
|---|---|---|---|---|
| $C_1$ | 3,3-dimethylbutyl | 132 - 133 | 0.62 | $C_{11}H_{22}N_2O_3$ |
| $C_2$ | 4,4-dimethylpentyl | waxy solid | 0.53 | $C_{12}H_{24}N_2O_3$ |
| $C_3$ | 3,3-methylethylpentyl | 124 - 126 | 0.66 | $C_{13}H_{26}N_2O_3$ |
| $C_4$ | Cycloheptyl | amorphous solid | 0.67 | $C_{12}H_{22}N_2O_3$ |

(*) eluent: n-Butanol-Acetic Acid-$H_2O$ (5-2-2/V:V)

Example 4

Preparation of D-4-(3-chlorobenzoylamino)-5-(3,3-dimethylbutylamino)-5-oxopentanoic acid (Compound D-1 in Table D).

23.0g (0.1 moles) of D-4-amino-5-(3,3-dimethylbutyl-5-oxopentanoic acid (Compound C1) were suspended in 300 ml of water and then dissolved under agitation by the addition of 10.6g (0.1 moles) of sodium carbonate. 17.5 g (0.1 moles) of 3-chlorobenzoyl chloride were then added over a period of 1 hour at 0 ° C under agitation.

The mixture was left to react for 12 hours.

It was acidified to Congo red with dilute HCl and the precipitate thus formed was filtered. Crystallisation was carried out with isopropyl ether/ethyl acetate (3:2).

M.P.: 129-132 ° C. TLC (isoamyl alcohol-acetone-$H_2O$: 5/2/1/): Rf 0.78.

29.5 g were obtained (mw 368.9). Yield 80%.

Rotatory power: $[alpha]^{20}_D$ = 1.90 ° C (c = 4.0% in methanol).

All the compounds of formula I (see scheme) were synthesised by the same method.

Table D below gives the compounds thus obtained with some of their identifying characteristics, as well as the yields obtained.

In order to check the optical purity of the compounds produced by the stereo-conservative synthesis described above, HPLC analysis was carried out with the use of a Macherey-Nagel Resolvosil/-BSA 7 column as the stationary phase and 0.1M phosphate buffer (pH 8) + 5% n-propanol as the mobile phase.

The compounds given in Table D showed an optical purity of more than 95% in all cases. The retention times are given in the table.

TABLE D: derivatives (D-Series) having the formula:

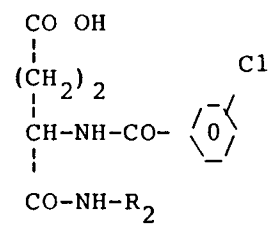

| COM-POUNDS | R2 | MELTING POINT (°C) | CRYSTALLISATION SOLVENT | HPLC RETENTION TIME (minutes) | ROTATORY POWER $[alpha]_D$ in methanol | FORMULA | YIELD | NOTE |
|---|---|---|---|---|---|---|---|---|
| D1 | 3,3-dimethyl-butyl | 129-132 | Isopr. ether/Et. Acetate (3:2) | 7.9 | +1.9 | $C_{18}H_{25}ClN_2O_4$ | 80 | - |
| D2 | 4,4-dimethyl pentyl | 155(dec.) | Alcohol-$H_2O$ (1:1) | 29.7 | +1.0 | $C_{19}H_{27}ClN_2O_4$ | 67 | (1) |
| D3 | 3,3-methyl-ethylpentyl | 128-131 | Isopr. ether/Et. Acetate (3:2) | 33.5 | +2.6 | $C_{20}H_{29}ClN_2O_4$ | 75 | - |
| D4 | Cycloheptyl | 140-145 | Isopr. ether/Et. Acetate (1:2) | 11.9 | -1.5 | $C_{19}H_{25}ClN_2O_4$ | 83 | - |
| D5 | 3,3-dimethyl-butyl | 128-131 | Isopr.ether/Et. Acetate (3:2) | 7.0 | -2.0 | $C_{18}H_{25}ClN_2O_4$ | - | (2) |
| D6 | Cycloheptyl | 115-125 | - | 10.3 | +1.7 | $C_{19}H_{25}ClN_2O_4$ | - | (2) |

(1) isolated as the calcium salt.
(2) L-series shown for comparison.

The anti-acid-secretion activities of the compounds of the invention, displayed through an antigastrin mechanism, were analysed by means of a series of pharmacological tests, both in vitro and in vivo, and are documented below.

Studies on binding to isolated guinea-pig stomach glands.

The capacity of some of the compounds which are the subject of the invention to inhibit the binding of pentagastrin-[beta-alanyl-3-3H(N)] to the gastrin receptor sites on the parietal cell membranes of the fundic mucosa of a guinea-pig stomach was evaluated by comparison with the displacement of pentagastrin induced by proglumide, which is a reference antigastrin compound, by lorglumide, which is a powerful CCK antagonist mentioned above, and by the compound D5 which is the optical antipode (the L-series enantiomer) of the compound D1.

Male guinea pigs weighing about 400-500g were used. After the guinea pig had been killed, its stomach was removed and placed in oxygenated SPB buffer at 37 °C (SPB = saline phosphate buffer: 149.6 mM NaCl, 3 mM $K_2HPO_4$, 0.64 mM $NaH_2PO_4$, pH 7.3).

After washing in the buffer, the stomach was placed on a petri dish and the antral part discarded.

The mucosa of the remaining part was removed from the underlying muscular part and was then chopped and transferred to a flask containing a collagenase digestion solution, 0.1% BSA and 0.2% glucose. The solution was incubated at 37 °C for 10 minutes under agitation and a stream of $0_2$.

During the incubation period, the tissue was passed several times through a series of pipettes with decreasing diameters in order to facilitate its digestion. At the end of the period, the tissue was removed and centrifuged at 1000 rpm. The supernatant liquid resulting from the centrifuging was discarded and the pellet washed several times with a washing buffer containing EPTA to remove the collagenase.

The glands were then isolated from the undigested tissue by conventional techniques, suspended in Hepes buffer for binding and incubated together with a radioactive tracer and the compounds under test for 30 minutes at 25 °C.

After the supernatant liquid had been removed by centrifuging, the radioactivity associated with the pellet was determined with a liquid scintillator. The specific binding was determined as the difference between the binding in the absence and in the presence of $10^{-6}$ M pentagastrin.

The results obtained are shown in Table 1 which gives the IC50, that is, the concentration (in moles/litre) of the antagonist which can displace 50% of the pentagastrin tritiate from the receptor.

TABLE 1 : Inhibition of the binding of pentagastrin [beta-alanyl-3-3H(N)] - to isolated guinea-pig gastric glands

| COMPOUND | IC50 (moles/litre) | COMPOUNDS | IC50 (moles/litre) |
|---|---|---|---|
| Pentagastrin | $4.1 \times 10^{-10}$ | A17 | $4.5 \times 10^{-6}$ |
| A1 | $2.0 \times 10^{-6}$ | A18 | $5.8 \times 10^{-6}$ |
| A2 | $3.1 \times 10^{-6}$ | A19 | $4.1 \times 10^{-5}$ |
| A3 | $4.8 \times 10^{-6}$ | A20 | $6.8 \times 10^{-7}$ |
| A4 | $3.3 \times 10^{-6}$ | A21 | $6.9 \times 10^{-8}$ |
| A5 | $8.0 \times 10^{-8}$ | A22 | $2.9 \times 10^{-6}$ |
| A6 | $4.5 \times 10^{-6}$ | A23 | $6.6 \times 10^{-8}$ |
| A7 | $1.7 \times 10^{-5}$ | A24 | $2.3 \times 10^{-7}$ |
| A8 | $1.2 \times 10^{-5}$ | A25 | $7.3 \times 10^{-6}$ |
| A9 | $2.0 \times 10^{-7}$ | A26 | $8.4 \times 10^{-7}$ |
| A10 | $2.3 \times 10^{-7}$ | D1 | $5.3 \times 10^{-8}$ |
| A11 | $6.5 \times 10^{-7}$ | D2 | $3.4 \times 10^{-8}$ |
| A12 | $3.8 \times 10^{-6}$ | D3 | $5.8 \times 10^{-8}$ |
| A13 | $9.1 \times 10^{-8}$ | D4 | $2.3 \times 10^{-6}$ |
| A14 | $7.8 \times 10^{-8}$ | D5 (1) | $5.2 \times 10^{-7}$ |
| A15 | $4.4 \times 10^{-7}$ | D6 (1) | $6.7 \times 10^{-5}$ |
| A16 | $2.9 \times 10^{-7}$ | Proglumide (1) | $5.7 \times 10^{-4}$ |
| | | Lorglumide (1) | $1.1 \times 10^{-5}$ |

Note (1): these compounds were tested for comparison.

It can be seen from the data given in the table that the compounds which are the subject of the invention antagonise the binding of pentagastrin by 50% in molar concentrations of between $10^{-5}$ and $10^{-8}$, depending on their structures.

The most active compounds achieve this activity at a molar concentration of around $5 \times 10^{-8}$ and thus about 100 times greater than that of the specific antagonist (cold pentagastrin), thus showing a high

specificity of action. Proglumide, which is an antigastrin reference drug, is approximately 10000 times less active than the most powerful compounds of the series whilst lorglumide, which is a specific antagonist of CCK, is about 200 times less active. The compound D5, which is an L-series derivative, is about 10 times less active than the corresponding compound D1 which is its D-series enantiomer.

Studies on binding to cerebral cortex membranes of mice

The displacement capacities of some of the compounds of the invention which were most active in the experimental model described above were to be evaluated but, in this case, with the use of the central gastrin receptors as the substrate. Cerebral cortex membranes were therefore used and pentagastrin tritiate was again used as the ligand.

Mouse cortex tissue was homogenised cold in 20 volumes of tris buffer (pH 7.7). After washing and centrifuging, the final pellet was resuspended in 20 volumes of binding buffer containing, inter alia, TRIS, BSA and bacitracin. 0.4 ml of the membrane thus obtained were then incubated with a radioactive tracer and with the compounds under test for 40 minutes at 37 °C.

After the supernatant liquid had been removed by centrifuging, the radioactivity associated with the pellet was determined, again with a liquid scintillator. The specific binding is the difference between the binding in the absence and in the presence of $10^{-6}$ M pentagastrin.

The results obtained are shown in Table 2. Again, this gives the IC50s of the compounds tested with the meaning as given above.

It can be seen from the data given in Table 2 that the most active of the compounds of the invention have IC50s of the order of magnitude of $10^{-8}$ M. Their relative potencies are unchanged from those described above for binding to the gastric glands. In fact, the most active compounds are about 100 times less powerful than the specific antagonist (pentagastrin) and about 100 times more powerful than the CCK-antagonist, lorglumide. In this case, the D-series derivatives (compound D1) are again about 10 times more active than the corresponding L-enantiomers (compound D5). As well as helping to show that the central and peripheral gastrin receptors seem to have a common structure, these results may be of practical interest since they predict a possible favourable use for the products of the invention in the treatment of cerebral disorders attributable to imbalances in the physiological neuron levels of gastrin.

TABLE 2 : Inhibition of the binding of pentagastrin [beta-alanyl-3-3H(N)] - to mouse cortical membranes.

| COMPOUND | IC50 (moles/litre) | COMPOUNDS | IC50 (moles/litre) |
|---|---|---|---|
| Pentagastrin | $7.8 \times 10^{-10}$ | A17 | $1.7 \times 10^{-5}$ |
| A1 | $6.7 \times 10^{-6}$ | A20 | $8.3 \times 10^{-7}$ |
| A3 | $7.5 \times 10^{-6}$ | A21 | $8.0 \times 10^{-8}$ |
| A5 | $9.6 \times 10^{-8}$ | A22 | $2.4 \times 10^{-6}$ |
| A8 | $3.9 \times 10^{-5}$ | A23 | $2.3 \times 10^{-7}$ |
| A9 | $2.2 \times 10^{-7}$ | A24 | $2.9 \times 10^{-7}$ |
| A10 | $1.8 \times 10^{-7}$ | D1 | $6.1 \times 10^{-8}$ |
| A12 | $6.4 \times 10^{-6}$ | D2 | $5.9 \times 10^{-8}$ |
| A13 | $9.2 \times 10^{-8}$ | D5 (1) | $6.6 \times 10^{-7}$ |
| A14 | $9.3 \times 10^{-8}$ | Proglumide (1) | $2.2 \times 10^{-4}$ |
| | | Lorglumide (1) | $6.1 \times 10^{-6}$ |

Note (1): these compounds were tested for comparison.

In order to confirm the antigastrin activity shown by these in vitro studies, the compounds of the inventon were also studied in vivo, through the experimental model described below.

Antisecretive activity in anaesthetised rats

The antisecretive activity was determined in rats with the use of male animals weighing about 200g, anaesthetised with urethane. Gastric secretion was stimulated with pentagastrin. K.S. Lai's method (Gut 5, (1964), 327-341) was used, with slight modifications.

After tracheotomy, oesophageal and duodenal cannulae were inserted. A tepid solution (37°C) of 0.25 mM NaOH was perfused through the stomach by means of a peristaltic pump at a constant flow rate of 1 ml/minute. After stabilisation for 20 minutes, the dose of the stimulant, indicated in Table 3, dissolved in a physiological solution, was perfused for 120 minutes at a rate of 0.95 ml/hour. After 60 minutes of perfusion (basic stimulation), the products under test were administered intravenously (I.V.) in boli whilst the perfusion of the stimulant continued for a further 60 minutes. The acid secretion was recorded continuously as a function of time.

The activity of the product was evaluated as the reduction in the secreted acidity after administration of the product as a percentage of the basic acidity measured during the first 60 minutes of collection during which only pentagastrin was present.

The antagonistic compounds tested were administered in different doses so that an ID50 (that is the dose (in mg/Kg I.V.) which can inhibit the effect of the pentagastrin by 50% could be calculated.

The results thus obtained are given in the following table in which the activities of the compounds are expressed as ID50s under the stimulus of pentagastrin at 30 mcg/Kg/h.

TABLE 3 : Antagonistic activity (ED50 mg/kg IV) towards gastric acid secretion induced by pentagastrin (30 mcg/kg/h)

| | | | |
|---|---|---|---|
| COMPOUND A1 | 56 | COMPOUND A18 | 58 |
| COMPOUND A2 | 48 | COMPOUND A19 | 98 |
| COMPOUND A3 | 54 | COMPOUND A20 | 44 |
| COMPOUND A4 | 61 | COMPOUND A21 | 34 |
| COMPOUND A5 | 35 | COMPOUND A22 | 41 |
| COMPOUND A6 | 55 | COMPOUND A23 | 40 |
| COMPOUND A7 | 93 | COMPOUND A24 | 42 |
| COMPOUND A8 | IN (>100 ) | COMPOUND A25 | 74 |
| COMPOUND A9 | 34 | COMPOUND A26 | 52 |
| COMPOUND A10 | 37 | COMPOUND D1 | 28 |
| COMPOUND A11 | 53 | COMPOUND D2 | 34 |
| COMPOUND A12 | 58 | COMPOUND D3 | 30 |
| COMPOUND A13 | 33 | COMPOUND D4 | 64 |
| COMPOUND A14 | 39 | COMPOUND D5 | 89 |
| COMPOUND A15 | 43 | COMPOUND Proglumide | 500 |
| COMPOUND A16 | 37 | COMPOUND Lorglumide | IN (>100) |
| COMPOUND A17 | 65 | | |

The antigastrin activity was particularly favourable in the case of the glutamic acid derivatives (n = 2), when $R_1$ was 3-chlorophenyl or 3,5-chlorophenyl, or when the alkyl group in $R_2$ had a length of at most 4 or 5 carbon atoms and was sufficiently branched at its (distal) end remote from the secondary amide group.

It should be noted that the compounds of the invention which were most active in this experimental model are about 15 times more active than the reference antigastrin compound, proglumide. It is also interesting to note that the CCK antagonist, lorglumide, was completely inactive up to a dose of 100 mg/Kg.

The action of these compounds against gastric secretion is specifically linked to their antigastrin activity. In fact they have no anticholinergic or antihistamine activity (anti $H_2$) since they were completely inactive when carbachol (30mcg/Kg/h) or histamine (2.3 mg/Kg/h) was used as the stimulant in the experimental model described above.

The activity of the compounds of the invention seems even more remarkable if one takes account of their low toxicity. For example, the compound D1 has an LD50 IV in mice of approximately 600mg/Kg. The toxic dose (LD50) in this case is therefore about 20 times greater than the antigastrin dose (ID50).

Anticholecystokinin (anti-CCK) activity

In order to check the hypothesis that the molecular conformation of the subject compounds is suitable to give them a specific antagonistic activity towards gastrin and not towards CCK, the anticontracturant activity of some of the compounds of the invention was tested on guinea-pig gall bladders stimulated in vitro by CCK-8.

A longitudinal strip of guinea-pig gall bladder was placed in a bath for isolated organs in the presence of Krebs at a temperature of 32 °C, continuously oxygenated by an oxygen-$CO_2$ mixture (95-5 V/V).

The isometric contractions were detected by means of a force transducer and recorded.

The gall bladder was contracted with the use of a 10ng/ml concentration of CCK-8; the antagonistic activity of the compounds towards the contracturant effect of the CCK was determined with the use of different concentrations and the IC50 value, that is, the concentration in mcg/ml of the compound which is able to antagonise the contracturant effect of the CCK by 50%, thus determined.

The results obtained are set out in the table below which gives the compounds tested and the IC50s which were calculated by the regression method on a set of at least three tests for each compound investigated.

Table 4

| Anti-CCK activity (concentration 10 ng/ml) expressed as the IC50 in mcg/ml on guinea-pig gall bladder in vitro. | |
|---|---|
| COMPOUND | IC50 ACTIVITY (mcg/ml) |
| A1 | > 100 |
| A7 | > 100 |
| A13 | 32 |
| A20 | > 100 |
| D1 | 83 |
| Lorglumide | 0.06 |

An examination of the table shows that the compounds claimed antagonise the contracturant activity of CCK-8 at a concentration such that the contracturant activity of the most active of the derivatives tested, the compound A13, was about 500 times greater than that of lorglumide, which shows that they have little or no anti-CCK activity.

The inhibiting effect on the rate of pentagastrin-induced growth of normal and tumorous colic cells

Gastrin has a trophic effect on the digestive epithelium. The chronic administration of gastrin (or pentagastrin, the biologically active part of the physiological hormone) to rats thus causes hyperplasia of the fundic mucosa and of the colic mucosa. Recently it has been shown that gastrin stimulates the growth of a transplantable mouse colic tumour produced by chemical induction (Winsell et al. -Surgical Forum, 33, 384 (1982).

On the basis of this premise, it was desired to investigate whether the specific gastrin antagonists of the invention could antagonise the growth of experimentally induced gastrointestinal tumours.

A gastrin antagonist of the invention which was one of the most active in the tests described above, that is the compound A-5, was selected for this purpose. Male mice weighing 20-25g were inoculated subcutaneously in the interscapular region with a suspension of 8 x $10^4$ tumour cells of a mouse colic adenocarcinoma.

5 groups each of 10 animals were used, that is : one control group of animals, one group of animals treated with 250 mcg/ml of pentagastrin twice a day and two groups of animals treated with the compound A-5 in doses of 15 and 30 mg/Kg i.p. respectively, twice a day, plus pentagastrin in the manner described above.

After 20 days, the animals were killed and the fundic mucosa and the tumours were removed, weighed and extracted in order to determine their DNA content. The results obtained are given in the following table.

Table 6 : Antagonistic activity of the compound A-5 on pentagastrin-induced colic tumorous growth

| GROUPS | No. ANIM. | WEIGHT OF THE FUNDIC MUCOSA (mg) | student t | FUNDIC DNA (mg) | student t | WEIGHT OF THE COLIC TUMOUR (mg) | student t | TUMOROUS DNA (mg) | student t |
|---|---|---|---|---|---|---|---|---|---|
| A) :Control | 10 | 18.0 ± 3.5 | - | 0.23 ± 0.03 | - | 521.1 ± 29 | - | 2.76 ± 0.23 | - |
| B) :Pentagastrin (PEG) | 10 | 36.0 ± 4.2 | 10.4 (**) | 0.44 ± 0.03 | 15.7 (**) | 779.7 ± 27 | 20.0(**) | 4.48 ± 0.29 | 8.34 (**) |
| C) :PEG+A-5 (15mg/Kg-2 x die | 10 | 25.1 ± 4.7 | vs A: 3.7(*) vs B: 5.4(**) | 0.28 ± 0.02 | vs A: 4.11(**) vs B: 14.6(**) | 579.0 ± 40 | vs A: 3.5(*) vs B: 12.0(**) | 3.26 ± 0.49 | vs A: 2.9(*) vs B: 2.9(*) |
| D) :PEG+A-5 (30mg/Kg-2 x die | 10 | 19.9 ± 2.1 | vs A: 1.5 vs B: 10.6 (**) | 0.23 ± 0.05 | vs A: 0.0 vs B: 12.1(**) | 559.5 ± 43 | vs A: 2.1 vs B: 13.6(**) | 2.87 ± 0.2 | vs A: 1.13 vs B 7.95 (**) |

(*) : $P < 0.01$
(**) : $P < 0.001$

The data in Table 6 show that pentagastrin induces a significant hyperplasia of the fundic mucosa and a significant increase in the weight of the colic tumour and in its DNA content. The compound A-5 can inhibit both of the aforesaid trophic effects of pentagastrin in a significant and dose-dependent manner.

## Claims

### Claims for the following Contracting States : AT, BE, CH, DE, FR, GB, IT, LI, NL

1. Glutamic and aspartic acid derivatives having the general formula:

CO OH
¦
$(CH_2)n$
¦
(*) $CH-NH-CO-R_1$ (I)
¦
$CO-NH-R_2$

in which n is 1 or 2, $R_1$ is a phenyl group mono- or di-substituted with chloro, methyl or nitro groups such that hydrogen or the nitro group may be present in positions 2 and 6 of the phenyl group and, independently, hydrogen, chloro or the methyl group may be present in positions 3 and 5, and in which $R_2$ is a linear or branched alkyl chain or a cyclo-alkyl group containing from 5 to 9 carbon atoms, the stereochemistry at the chiral centre marked with an asterisk in formula (I) being DL or D, and their pharmaceutically acceptable salts.

2. A derivative of glutamic acid according to Claim 1 in which $R_1$ is selected from the group consisting of 3-chlorophenyl and 3,5-dichlorophenyl, $R_2$ is selected from the group consisting of 3,3-dimethylbutyl, 4,4-dimethylpentyl and 3,3-methyl-ethylpentyl, the stereochemistry of the chiral centre being DL or D, and their pharmaceutically acceptable salts.

3. Compounds according to Claim 1, that is:
DL-4-[(3-chlorobenzoyl)-amino]-5-(pentylamino)-5-oxopentanoic acid;
DL-4-[(3-chlorobenzoyl)-amino]-5-(3-methylbutylamino)-5-oxopentanoic acid;
DL-4-[(3-chlorobenzoyl)-amino]-5-(hexylamino)-5-oxopentanoic acid;
DL-4-[(3-chlorobenzoyl)-amino]-5-(3,3-dimethylbutylamino)-5-oxopentanoic acid;
DL-4-[(3-chlorobenzoyl)-amino]-5-[3-methylpentylamino)-5-oxopentanoic acid;
DL-4-[(3-chlorobenzoyl)-amino]-5-(cyclohexylamino)-5-oxopentanoic acid;
DL-4-[(3-chlorobenzoyl)-amino]-5-(heptylamino)-5-oxopentanoic acid;
DL-4-[(3-chlorobenzoyl)-amino]-5-(4,4-dimethylpentyl)-5-oxopentanoic acid;
DL-4-[(3-chlorobenzoyl)-amino]-5-(3,3-dimethylpentylamino)-5-oxopentanoic acid;
DL-4-[(3-chlorobenzoyl)-amino]-5-(3-ethylpentyl)-5-oxopentanoic acid;
DL-4-[(3-chlorobenzoyl)-amino]-5-(cycloheptylamino)-5-oxopentanoic acid;
DL-4-[(3-chlorobenzoyl)-amino]-5-(4-methylpentylamino)--5-oxopentanoic acid;
DL-4-[(3-chlorobenzoyl)-amino]-5-(3,3-methylethylpentylamino)-5-oxopentanoic acid;
DL-4-[(3-chlorobenzoyl)-amino]-5-(3,3-diethylpentylamino)-5-oxopentanoic acid;
DL-4-[(3-chlorobenzoyl)-amino]-5-(2-(dicyclo[2.2.1]-heptyl)-ethylamino)-5-oxopentanoic acid;
DL-4-[(3-chlorobenzoyl)-amino]-5-(3,3-methylethyl-4-methylpentylamino)-5-oxopentanoic acid;
DL-4-[(3,5-dichlorobenzoyl)amino]-5-(3,3-dimethylbutylamino)-5-oxopentanoic acid;
DL-3-[(3-chlorobenzoyl)-amino]-4-(3,3-methylethylpentylamino)-4-oxobutanoic acid;
DL-3-[(3-chlorobenzoyl)-amino]-4-(cycloheptylamino)-4-oxobutanoic acid;
D-4-[(3-chlorobenzoyl)-amino]-5-(3,3-dimethylbutylamino)-5-oxopentanoic acid;
D-4-[(3-chlorobenzoyl)-amino]-5-(4,4-dimethylpentylamino)-5-oxopentanoic acid;
D-4-[(3-chlorobenzoyl)-amino]-5-(3,3-methylethylpentylamino)-5-oxopentanoic acid;
D-4-[(3-chlorobenzoyl)-amino]-5-(cycloheptylamino)-5-oxopentanoic acid;
and their pharmaceutically acceptable salts.

**4.** A pharmaceutical composition including at least one of the compounds according to any one of Claims 1 to 3 or a pharmaceutically acceptable salt thereof as the active agent and a pharmaceutically acceptable vehicle.

**5.** The use of glutamic and aspartic acid derivatives according to Claim 1 or a pharmaceutically acceptable salt thereof for the preparation of a medicament for the therapeutic treatment of disorders induced by hypergastrinaemia.

**6.** The use of a derivative according to Claim 1 for the preparation of a medicament for the therapeutic treatment of ulcers.

**7.** The use of a derivative according to Claim 1 for the preparation of a medicament for the therapeutic treatment of tumorous conditions sustained by gastrin and by other bioactive polypeptides related thereto.

**8.** The use of a derivative according to Claim 1 for the preparation of a medicament for the treatment of pathological conditions of the CNS linked to an imbalance in the physiological neuron levels of gastrin or other bioactive polypeptides related thereto.

**9.** A method for the preparation of a glutamic acid or aspartic acid derivative of formula (I), in which n, $R_1$ and $R_2$ have the meanings given in Claim 1 and in which substituents at the chiral centre (marked with an asterisk) have the DL conformation, characterised in that it comprises the steps of:
a) reacting an internal anhydride of formula (IV):

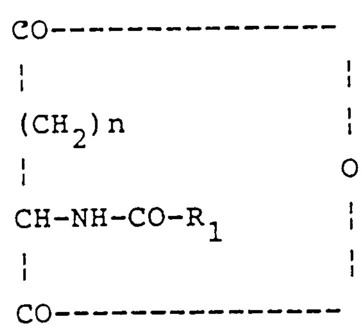

(IV)

in which n and $R_1$ have the meanings given above, with a primary amine of formula $R_2$-$NH_2$, in which $R_2$ has the meaning given above, in a molar ratio of from 1 to 5, at a temperature of from -10 °C to 10 °C, and recovering the compounds (I) from the reaction mass.

**10.** A method for the preparation of a glutamic acid or aspartic acid derivative of formula (I) in which n, $R_1$ and $R_2$ have the meanings given in Claim 1, and in which the substituents at the chiral centre (marked with an asterisk in formula (I)) have the (D) conformation, characterised in that it comprises the following stereo-conservative steps:
a) reacting the gamma-benzyl ester of N-carbobenzoxy-D-glutamic acid with an amine of formula $H_2$-$NR_2$, in which $R_2$ has the meaning given above, by the mixed anhydride method, at a temperature of between -15° and +15° and in an inert anhydrous solvent and recovering the compounds (III) from the reaction mass;

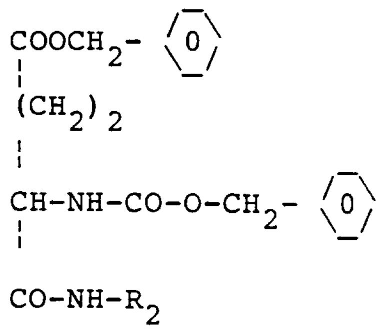

(III)

b) debenzylating and decarbobenzoxylating the compound of formula (III) dissolved in an inert solvent, such as methanol, by reacting it with hydrogen at ambient temperature and pressure in the presence of a catalytically effective quantity of hydrogenation catalyst and recovering the compounds (II) from the reaction mass;

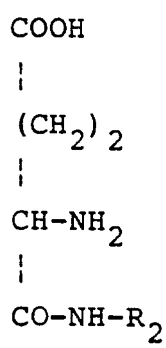

(II)

c) reacting the derivatives of formula (II) under Schotten-Baumann conditions with an equimolecular quantity of an acyl chloride of formula $R_1$-CO-Cl, in which $R_1$ has the meaning given in Claim 1, at a temperature of from 0 to 15 °C and recovering the derivatives of formula (I) with the D conformation from the reaction mass.

**Claims for the following Contracting State : ES**

**1.** A method for the preparation of a glutamic acid or aspartic acid derivative of formula (I):

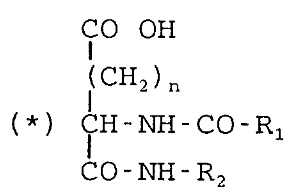

(I)

in which n is 1 or 2, $R_1$ is a phenyl group mono- or di-substituted with chloro, methyl or nitro groups such that hydrogen or the nitro group may be present in positions 2 and 6 of the phenyl group and, independently, hydrogen, chloro or the methyl group may be present in positions 3 and 5, and in which $R_2$ is a linear or branched alkyl chain or a cyclo-alkyl group containing from 5 to 9 carbon atoms, and their pharmaceutically acceptable salts,
and in which substituents at the chiral centre (marked with an asterisk) have the DL conformation, characterised in that it comprises the steps of:

a) reacting an internal anhydride of formula (IV):

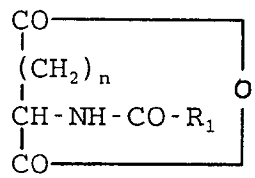

(IV)

in which n and $R_1$ have the meanings given above, with a primary amine of formula $R_2$-$NH_2$, in which $R_2$ has the meaning given above, in a molar ratio of from 1 to 5, at a temperature of from -10 °C to 10 °C, and recovering the compounds (I) from the reaction mass.

**2.** A method for the preparation of a glutamic acid or aspartic acid derivative of formula (I) in which n, $R_1$ and $R_2$ have the meanings given in Claim 1, and in which the substituents at the chiral centre (marked with an asterisk in formula (I)) have the (D) conformation, characterised in that it comprises the following stereo-conservative steps:

a) reacting the gamma-benzyl ester of N-carbobenzoxy-D-glutamic acid with an amine of formula $H_2$-$NR_2$, in which $R_2$ has the meaning given above, by the mixed anhydride method, at a temperature of between -15° and +15° and in an inert anhydrous solvent and recovering the compounds (III) from the reaction mass,

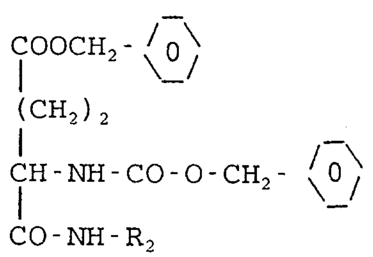

(III)

b) debenzylating and decarbobenzoxylating the compound of formula (III) dissolved in an inert solvent, such as methanol, by reacting it with hydrogen at ambient temperature and pressure in the presence of a catalytically effective quantity of hydrogenation catalyst and recovering the compounds (II) from the reaction mass,

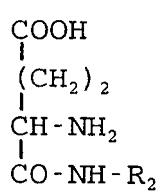

(II)

c) reacting the derivatives of formula (II) under Schotten-Baumann conditions with an equimolecular quantity of an acyl chloride of formula $R_1$-CO-C1, in which $R_1$ has the meaning given in Claim 1, at a temperature of from 0 to 15 °C and recovering the derivatives of formula (I) with the D conformation from the reaction mass.

**3.** A method for the preparation of a compound according to Claim 1 or 2, wherein $R_1$ is selected from the group consisting of 3-chlorophenyl and 3,5-dichlorophenyl, $R_2$ is selected from the group consisting of 3,3-dimethylbutyl, 4,4-dimethylpentyl and 3,3-methyl-ethylpentyl.

**4.** A method according to Claim 1 for the preparation of a derivative selected from:
DL-4-[(3-chlorobenzoyl)-amino]-5-(pentylamino)-5-oxopentanoic acid;
DL-4-[(3-chlorobenzoyl)-amino]-5-(3-methylbutylamino)-5-oxopentanoic acid;
DL-4-[(3-chlorobenzoyl)-amino]-5-(hexylamino)-5-oxopentanoic acid;
DL-4-[(3-chlorobenzoyl)-amino]-5-(3,3-dimethylbutylamino)-5-oxopentanoic acid;
DL-4-[(3-chlorobenzoyl)-amino]-5-[3-methylpentylamino)-5-oxopentanoic acid;
DL-4-[(3-chlorobenzoyl)-amino]-5-(cyclohexylamino)-5-oxopentanoic acid;
DL-4-[(3-chlorobenzoyl)-amino]-5-(heptylamino)-5-oxopentanoic acid;
DL-4-[(3-chlorobenzoyl)-amino]-5-(4,4-dimethylpentyl)-5-oxopentanoic acid;
DL-4-[(3-chlorobenzoyl)-amino]-5-(3,3-dimethylpentyl-amino)-5-oxopentanoic acid;
DL-4-[(3-chlorobenzoyl)-amino]-5-(3-ethylpentyl)-5-oxopentanoic acid;
DL-4-[(3-chlorobenzoyl)-amino]-5-(cycloheptylamino)-5-oxopentanoic acid;
DL-4-[(3-chlorobenzoyl)-amino]-5-(4-methylpentylamino)--5-oxopentanoic acid;
DL-4-[(3-chlorobenzoyl)-amino]-5-(3,3-methylethylpentylamino)-5-oxopentanoic acid;
DL-4-[(3-chlorobenzoyl)-amino]-5-(3,3-diethylpentylamino)-5-oxopentanoic acid;
DL-4-[(3-chlorobenzoyl)-amino]-5-(2-(dicyclo[2.2.1]-heptyl)-ethylamino)-5-oxopentanoic acid;
DL-4-[(3-chlorobenzoyl)-amino]-5-(3,3-methylethyl-4-methylpentylamino)-5-oxopentanoic acid;
DL-4-[(3,5-dichlorobenzoyl)amino]-5-(3,3-dimethylbutylamino)-5-oxopentanoic acid;
DL-3-[(3-chlorobenzoyl)-amino]-4-(3,3-methylethylpentylamino)-4-oxobutanoic acid;
DL-3-[(3-chlorobenzoyl)-amino]-4-(cycloheptylamino)-4-oxobutanoic acid;

**5.** A method according to Claim 2 for the preparation of a compound selected from:
D-4-[(3-chlorobenzoyl)-amino]-5-(3,3-dimethylbutylamino)-5-oxopentanoic acid;
D-4-[(3-chlorobenzoyl)-amino]-5-(4,4-dimethylpentylamino)-5-oxopentanoic acid;
D-4-[(3-chlorobenzoyl)-amino]-5-(3,3-methylethylpentylamino)-5-oxopentanoic acid;
D-4-[(3-chlorobenzoyl)-amino]-5-(cycloheptylamino)-5-oxopentanoic acid;
and their pharmaceutically acceptable salts.

**Patentansprüche**

**Patentansprüche für folgende Vertragsstaaten : AT, BE, CH, DE, FR, GB, IT, LI, NL**

**1.** Glutaminsäure- und Asparaginsäure-Derivate der allgemeinen Formel

CO OH
|
$(CH_2)n$
|
(*) $CH-NH-CO-R_1$ (I)
|
$CO-NH-R_2$

in welcher n 1 oder 2 bedeutet, $R_1$ eine Phenylgruppe, die mit Chlor-, Methyl- oder Nitrogruppen mono- oder disubstituiert ist, darstellt, wobei Wasserstoff oder die Nitrogruppe in den Positionen 2 und 6 der Phenylgruppe und, unabhängig voneinander, Wasserstoff, Chlor oder die Methylgruppe in den Positionen 3 und 5 vorliegen können, und in welcher $R_2$ eine lineare oder verzweigte Alkylkette oder eine Cycloalkylgruppe mit 5 bis 9 Kohlenstoffatomen bedeutet, wobei die Stereochemie an dem mit einem Asterix in Formel (I) markierten chiralen Zentrum DL oder D ist, sowie deren pharmazeutisch verwendbare Salze.

**2.** Ein Derivat der Glutaminsäure nach Anspruch 1, worin $R_1$ ausgewählt ist aus der Gruppe bestehend aus 3-Chlorphenyl und 3,5-Dichlorphenyl und $R_2$ ausgewählt ist aus der Gruppe bestehend aus 3,3-Dimethylbutyl, 4,4-Dimethylpentyl und 3,3-Methylethylpentyl, wobei die Stereochemie des chiralen Zentrums DL oder D ist, sowie deren pharmazeutisch verwendbare Salze.

3. Verbindungen nach Anspruch 1, und zwar:
DL-4-[(3-Chlorbenzoyl)-amino]-5-(pentylamino)-5-oxopentansäure,
DL-4-[(3-Chlorbenzoyl)-amino]-5-(methylbutylamino)-5-oxopentansäure,
DL-4-[(3-Chlorbenzoyl)-amino]-5-(hexylamino)-5-oxopentansäure,
DL-4-[(3-Chlorbenzoyl)-amino]-5-(3,3-dimethylbutylamino)-5-oxopentansäure,
DL-4-[(3-Chlorbenzoyl)-amino]-5-(3-methylpentylamino)-5-oxopentansäure,
DL-4-[(3-Chlorbenzoyl)-amino]-5-(cyclohexylamino)-5-oxopentansäure,
DL-4-[(3-Chlorbenzoyl)-amino]-5-(heptylamino)-5-oxopentansäure,
DL-4-[(3-Chlorbenzoyl)-amino]-5-(4,4-dimethylpentylamino)-5-oxopentansäure,
DL-4-[(3-Chlorbenzoyl)-amino]-5-(3,3-dimethylpentylamino)-5-oxopentansäure,
DL-4-[(3-Chlorbenzoyl)-amino]-5-(3-ethylpentylamino)-5-oxopentansäure,
DL-4-[(3-Chlorbenzoyl)-amino]-5-(cycloheptylamino)-5-oxopentansäure,
DL-4-[(3-Chlorbenzoyl)-amino]-5-(4-methylpentylamino)-5-oxopentansäure,
DL-4-[(3-Chlorbenzoyl)-amino]-5-(3,3-methylethylpentylamino)-5-oxopentansäure,
DL-4-[(3-Chlorbenzoyl)-amino]-5-(3,3-diethylpentylamino)-5-oxopentansäure,
DL-4-[(3-Chlorbenzoyl)-amino]-5-(2-(dicyclo[2.2.1]-heptyl)-ethylamino)-5-oxopentansäure,
DL-4-[(3-Chlorbenzoyl)-amino]-5-(3,3-methylethyl-4-methylpentylamino)-5-oxopentansäure,
DL-4-[(3,5-Dichlorbenzoyl)-amino]-5-(3,3-dimethylbutylamino)-5-oxopentansäure,
DL-3-[(3-Chlorbenzoyl)-amino]-4-(3,3-methylethylpentylamino)-4-oxobutansäure,
DL-3-[(3-Chlorbenzoyl)-amino]-4-(cycloheptylamino)-4-oxobutansäure,
D-4-[(3-Chlorbenzoyl)-amino]-5-(3,3-dimethylbutylamino)-5-oxopentansäure,
D-4-[(3-Chlorbenzoyl)-amino]-5-(4,4-dimethylpentylamino)-5-oxopentansäure,
D-4-[(3-Chlorbenzoyl)-amino]-5-(3,3-methylethylpentylamino)-5-oxopentansäure,
D-4-[(3-Chlorbenzoyl)-amino]-5-(cycloheptylamino)-5-oxopentansäure,
und deren pharmazeutisch verwendbare Salze.

4. Eine pharmazeutische Zusammensetzung, die mindestens eine der Verbindungen nach einem der Ansprüche 1 bis 3 oder ein pharmazeutisch verwendbares Salz derselben als Wirkstoff und einen pharmazeutisch verwendbaren Träger enthält.

5. Die Verwendung von Glutaminsäure- und Asparaginsäure-Derivaten nach Anspruch 1 oder eines pharmazeutisch verwendbaren Salzes derselben zur Herstellung eines Arzneimittels zur therapeutischen Behandlung von Störungen, die durch Hypergastrinämie hervorgerufen sind.

6. Die Verwendung eines Derivates nach Anspruch 1 zur Herstellung eines Arzneimittels für die therapeutische Behandlung von Ulcera.

7. Die Verwendung eines Derivates nach Anspruch 1 zur Herstellung eines Arzneimittels für die therapeutische Behandlung von tumorösen Zuständen, die durch Gastrin oder andere damit verwandte bioaktive Polypeptide unterstützt sind.

8. Die Verwendung eines Derivates nach Anspruch 1 zur Herstellung eines Arzneimittels für die Behandlung von pathologischen Zuständen des CNS im Zusammenhang mit einem Ungleichgewicht in den physiologischen Neurongehalten von Gastrin oder anderen damit verwandten bioaktiven Polypeptiden.

9. Ein Verfahren zur Herstellung eines Glutaminsäure- oder Asparaginsäure-Derivates der Formel (I), worin n, $R_1$ und $R_2$ die in Anspruch 1 angegebenen Bedeutungen haben und worin Substituenten an dem chiralen Zentrum (mit einem Asterix markiert) die DL-Konfiguration aufweisen, dadurch gekennzeichnet, daß es folgende Stufen umfaßt:

a) Umsetzung eines internen Anhydrids der Formel (IV)

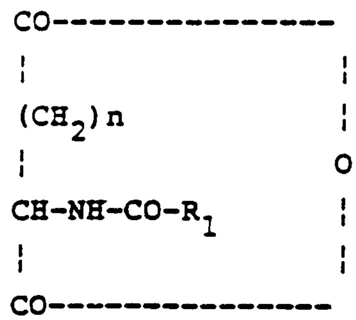

(IV)

worin n und $R_1$ die oben angegebene Bedeutung haben, mit einem primären Amin der Formel $R_2$-$NH_2$ , worin $R_2$ die oben angegebene Bedeutung hat, in einem Molverhältnis von 1 bis 5 bei einer Temperatur von -10 °C bis 10 °C und Gewinnung der Verbindungen (I) aus der Reaktionsmasse.

**10.** Ein Verfahren zur Herstellung eines Glutaminsäure- oder Asparaginsäure-Derivates der Formel (I), in welcher n, $R_1$ und $R_2$ die in Anspruch 1 angegebenen Bedeutungen haben und in welcher die Substituenten an dem chiralen Zentrum (mit einem Asterix in Formel (I) markiert) die (D)-Konfiguration haben, dadurch gekennzeichnet, daß es die folgenden stereokonservativen Stufen umfaßt:

a) Umsetzung des gamma-Benzylesters der N-Carbobenzoxy-D-glutaminsäure mit einem Amin der Formel $H_2$-$NR_2$, in welcher $R_2$ die oben angegebene Bedeutung hat, nach der Methode der gemischten Anhydride bei einer Temperatur zwischen -15 °C und +15 °C in einem inerten wasserfreien Lösungsmittel und Gewinnung der Verbindungen (III) aus der Reaktionsmasse;

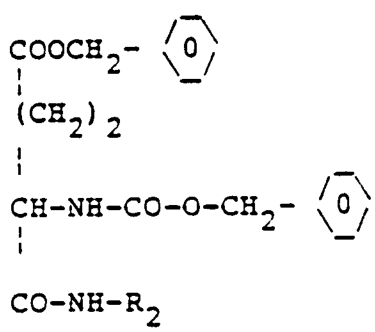

(III)

b) Debenzylierung und Decarbobenzoxylierung der Verbindung der Formel (III), die in einem inerten Lösungsmittel, wie Methanol, gelöst ist, durch Reaktion derselben mit Wasserstoff bei Raumtemperatur und Druck in Gegenwart einer katalytisch wirksamen Menge eines Hydrierungskatalysators und Gewinnung der Verbindungen (II) aus der Reaktionsmasse;

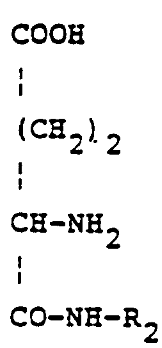

(II)

c) Umsetzung der Derivate der Formel (II) unter Schotten-Baumann-Bedingungen mit einer äquimolaren Menge eines Acylchlorids der Formel $R_1$-CO-Cl, in welcher $R_1$ die in Anspruch 1 angegebene Bedeutung hat, bei einer Temperatur von 0 bis 15 °C und Gewinnung der Derivate der Formel (I) mit der D-Konfiguration aus der Reaktionsmasse.

**Patentansprüche für folgenden Vertragsstaat : ES**

1. Verfahren zur Herstellung eines Glutaminsäure- und Asparaginsäure-Derivates der Formel (I):

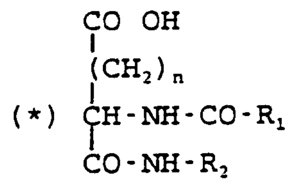

(I)

in welcher n 1 oder 2 bedeutet, $R_1$ eine Phenylgruppe, die mit Chlor-, Methyl- oder Nitrogruppen mono- oder disubstituiert ist, darstellt, wobei Wasserstoff oder die Nitrogruppe in den Positionen 2 und 6 der Phenylgruppe und, unabhängig voneinander, Wasserstoff, Chlor oder die Methylgruppe in den Positionen 3 und 5 vorliegen können, und in welcher $R_2$ eine lineare oder verzweigte Alkylkette oder eine Cycloalkylgruppe mit 5 bis 9 Kohlenstoffatomen bedeutet, sowie der pharmazeutisch verwendbaren Salze derselben,
wobei die Substituenten an dem chiralen Zentrum (mit einem Asterix markiert) die DL-Konfiguration aufweisen, dadurch gekennzeichnet, daß es folgende Stufen umfaßt:
a) Umsetzung eines internen Anhydrids der Formel (IV)

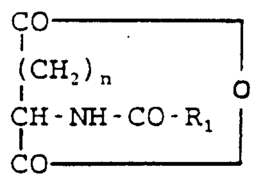

(IV)

worin n und $R_1$ die oben angegebene Bedeutung haben, mit einem primären Amin der Formel $R_2$-$NH_2$ , worin $R_2$ die oben angegebene Bedeutung hat, in einem Molverhältnis von 1 bis 5 bei einer Temperatur von -10 °C bis 10 °C und Gewinnung der Verbindungen (I) aus der Reaktionsmasse.

2. Ein Verfahren zur Herstellung eines Glutaminsäure- oder Asparaginsäure-Derivates der Formel (I), worin n, $R_1$ und $R_2$ die in Anspruch 1 angegebenen Bedeutungen haben und worin die Substituenten an dem chiralen Zentrum (mit einem Asterix in Formel (I) markiert) die (D)-Konfiguration aufweisen, dadurch gekennzeichnet, daß es die folgenden stereokonservativen Stufen umfaßt:
a) Umsetzung des gamma-Benzylesters der N-Carbobenzoxy-D-glutaminsäure mit einem Amin der Formel $H_2$-$NR_2$, in welcher $R_2$ die oben angegebene Bedeutung hat, nach der Methode der gemischten Anhydride bei einer Temperatur zwischen -15 °C und +15 °C in einem inerten wasserfreien Lösungsmittel und Gewinnung der Verbindungen (III) aus der Reaktionsmasse;

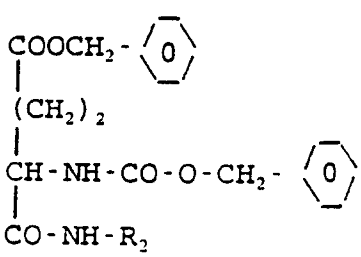

(III)

b) Debenzylierung und Decarbobenzoxylierung der Verbindung der Formel (III), die in einem inerten Lösungsmittel, wie Methanol, gelöst ist, durch Reaktion derselben mit Wasserstoff bei Raumtemperatur und Druck in Gegenwart einer katalytisch wirksamen Menge eines Hydrierungskatalysators und Gewinnung der Verbindungen (II) aus der Reaktionsmasse;

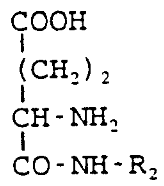

(II)

c) Umsetzung der Derivate der Formel (II) unter Schotten-Baumann-Bedingungen mit einer äquimolaren Menge eines Acylchlorids der Formel $R_1$-CO-Cl, in welcher $R_1$ die in Anspruch 1 angegebene Bedeutung hat, bei einer Temperatur von 0 bis 15 °C und Gewinnung der Derivate der Formel (I) mit der D-Konfiguration aus der Reaktionsmasse.

**3.** Ein Verfahren zur Herstellung einer Verbindung nach Anspruch 1 oder 2, worin $R_1$ ausgewählt ist aus der Gruppe bestehend aus 3-Chlorphenyl und 3,5-Dichlorphenyl und $R_2$ ausgewählt ist aus der Gruppe bestehend aus 3,3-Dimethylbutyl, 4,4-Dimethylpentyl und 3,3-Methylethylpentyl.

**4.** Ein Verfahren nach Anspruch 1 zur Herstellung eines Derivates, das ausgewählt ist aus:
DL-4-[(3-Chlorbenzoyl)-amino]-5-(pentylamino)-5-oxopentansäure,
DL-4-[(3-Chlorbenzoyl)-amino]-5-(3-methylbutylamino)-5-oxopentansäure,
DL-4-[(3-Chlorbenzoyl)-amino]-5-(hexylamino)-5-oxopentansäure,
DL-4-[(3-Chlorbenzoyl)-amino]-5-(3,3-dimethylbutylamino)-5-oxopentansäure,
DL-4-[(3-Chlorbenzoyl)-amino]-5-(3-methylpentylamino)-5-oxopentansäure,
DL-4-[(3-Chlorbenzoyl)-amino]-5-(cyclohexylamino)-5-oxopentansäure,
DL-4-[(3-Chlorbenzoyl)-amino]-5-(heptylamino)-5-oxopentansäure,
DL-4-[(3-Chlorbenzoyl)-amino]-5-(4,4-dimethylpentylamino)-5-oxopentansäure,
DL-4-[(3-Chlorbenzoyl)-amino]-5-(3,3-dimethylpentylamino)-5-oxopentansäure,
DL-4-[(3-Chlorbenzoyl)-amino]-5-(3-ethylpentylamino)-5-oxopentansäure,
DL-4-[(3-Chlorbenzoyl)-amino]-5-(cycloheptylamino)-5-oxopentansäure,
DL-4-[(3-Chlorbenzoyl)-amino]-5-(4-methylpentylamino)-5-oxopentansäure,
DL-4-[(3-Chlorbenzoyl)-amino]-5-(3,3-methylethylpentylamino)-5-oxopentansäure,
DL-4-[(3-Chlorbenzoyl)-amino]-5-(3,3-diethylpentylamino)-5-oxopentansäure,
DL-4-[(3-Chlorbenzoyl)-amino]-5-(2-(dicyclo[2.2.1]-heptyl)-ethylamino)-5-oxopentansäure,
DL-4-[(3-Chlorbenzoyl)-amino]-5-(3,3-methylethyl-4-methylpentylamino)-5-oxopentansäure,
DL-4-[(3,5-Dichlorbenzoyl)-amino]-5-(3,3-dimethylbutylamino)-5-oxopentansäure,
DL-3-[(3-Chlorbenzoyl)-amino]-4-(3,3-methylethylpentylamino)-4-oxobutansäure,
DL-3-[(3-Chlorbenzoyl)-amino]-4-(cycloheptylamino)-4-oxobutansäure.

**5.** Ein Verfahren nach Anspruch 2 zur Herstellung einer Verbindung, die ausgewählt ist aus:
D-4-[(3-Chlorbenzoyl)-amino]-5-(3,3-dimethylbutylamino)-5-oxopentansäure,
D-4-[(3-Chlorbenzoyl)-amino]-5-(4,4-dimethylpentylamino)-5-oxopentansäure,
D-4-[(3-Chlorbenzoyl)-amino]-5-(3,3-methylethylpentylamino)-5-oxopentansäure,
D-4-[(3-Chlorbenzoyl)-amino]-5-(cycloheptylamino)-5-oxopentansäure,
oder deren pharmazeutisch verwendbaren Salzen.

**Revendications**

**Revendications pour les Etats contractants suivants : AT, BE, CH, DE, FR, GB, IT, LI, NL**

**1.** Dérivés d'acides glutamique et aspartique de formule générale :

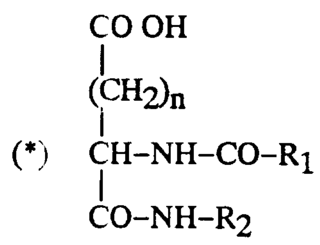

(I)

dans laquelle n vaut 1 ou 2, $R_1$ est un groupement phényle mono- ou disubstitué par des groupements chloro, méthyle ou nitro tels que l'atome d'hydrogène ou le groupement nitro peuvent être présents en position 2 et 6 du groupement phényle et, indépendamment, l'atome d'hydrogène, l'atome de chlore ou le groupement méthyle peut être présent aux positions 3 et 5, et dans laquelle $R_2$ représente une chaîne alkyle linéaire ou ramifiée ou un groupement cycloalkyle en $C_5$-$C_9$, la stéréochimie du centre chiral marquée par un astérisque dans la formule (I) étant DL ou D, et leurs sels pharmaceutiquement acceptables.

**2.** Dérivé d'acide glutamique selon la revendication 1, dans lequel $R_1$ est choisi dans le groupe constitué des groupements 3-chlorophényle et 3,5-dichlorophényle, $R_2$ est choisi dans le groupe constitué des groupements 3,3-diméthylbutyle, 4,4-diméthylpentyle, et 3,3-méthyléthylpentyle, la stéréochimie du centre chiral étant DL ou D, et ses sels pharmaceutiquement acceptables.

**3.** Composés selon la revendication 1, qui sont :
acide DL-4-[(3-chlorobenzoyl)amino]-5-(pentylamino)-5-oxopentanoïque ;
acide DL-4-[(3-chlorobenzoyl)amino]-5-(3-méthylbutylamino)-5-oxopentanoïque ;
acide DL-4-[(3-chlorobenzoyl)-amino]-5-(hexylamino)-5-oxopentanoïque ;
acide DL-4-[(3-chlorobenzoyl)-amino]-5-(3,3-diméthylbutylamino)-5-oxopentanoïque ;
acide DL-4-[(3-chlorobenzoyl)-amino]-5-(3-méthylpentylamino)-5-oxopentanoïque ;
acide DL-4-[(3-chlorobenzoyl)-amino]-5-(cyclohexylamino)-5-oxopentanoïque ;
acide DL-4-[(3-chlorobenzoyl)-amino]-5-(heptylamino)-5-oxopentanoïque ;
acide DL-4-[(3-chlorobenzoyl)-amino]-5-(4,4-diméthylpentyl)-5-oxopentanoïque ;
acide DL-4-[(3-chlorobenzoyl)-amino]-5-(3,3-diméthylpentylamino)-5-oxopentanoïque ;
acide DL-4-[(3-chlorobenzoyl)-amino]-5-(3-éthylpentyl)-5-oxopentanoïque ;
acide DL-4-[(3-chlorobenzoyl)-amino]-5-(cycloheptylamino)-5-oxopentanoïque ;
acide DL-4-[(3-chlorobenzoyl)-amino]-5-(4-méthylpentylamino)-5-oxopentanoïque ;
acide DL-4-[(3-chlorobenzoyl)-amino]-5-(3,3-méthyléthylpentylamino)-5-oxopentanoïque ;
acide DL-4-[(3-chlorobenzoyl)-amino]-5-(3,3-diéthylpentylamino)-5-oxopentanoïque ;
acide DL-4-[(3-chlorobenzoyl)-amino]-5-(2-(dicyclo[2.2.1]-heptyl)-éthylamino)-5-oxopentanoïque ;
acide DL-4-[(3-chlorobenzoyl)-amino]-5-(3,3-méthyléthyl-4-méthylpentylamino)-5-oxopentanoïque ;
acide DL-4-[(3,5-dichlorobenzoyl)-amino]-5-(3,3-diméthylbutylamino)-5-oxopentanoïque ;
acide DL-3-[(3-chlorobenzoyl)-amino]-4-(3,3-méthyléthylpentylamino)-4-oxobutanoïque ;
acide DL-3-[(3-chlorobenzoyl)-amino]-4-(cycloheptylamino)-4-oxobutanoïque ;
acide D-4-[(3-chlorobenzoyl)-amino]-5-(3,3-diméthylbutylamino)-5-oxopentanoïque ;
acide D-4-[(3-chlorobenzoyl)-amino]-5-(4,4-diméthylpentylamino)-5-oxopentanoïque.
acide D-4-[(3-chlorobenzoyl)-amino]-5-(3,3-méthyléthylpentylamino)-5-oxopentanoïque ;
acide D-4-[(3-chlorobenzoyl)-amino]-5-(cycloheptylamino)-5-oxopentanoïque ;
et leurs sels pharmaceutiquement acceptables.

**4.** Composition pharmaceutique comprenant au moins l'un des composés selon l'une quelconque des revendications 1 à 3, ou un sel pharmaceutiquement acceptable de celui-ci en tant que principe actif, et un véhicule pharmaceutiquement acceptable.

**5.** Utilisation de dérivés d'acides glutamique et aspartique selon la revendication 1 ou de sels pharmaceutiquement acceptables de ceux-ci pour la préparation d'un médicament pour le traitement thérapeutique de troubles provoqués par une hypergastrinémie.

**6.** Utilisation d'un dérivé selon la revendication 1 pour la préparation d'un médicament destiné au traitement thérapeutique d'ulcères.

**7.** Utilisation d'un dérivé selon la revendication 1 destiné à la préparation d'un médicament pour le traitement thérapeutique d'états tumoraux entretenus par la gastrine et par d'autres polypeptides bioactifs apparentés.

**8.** Utilisation d'un dérivé selon la revendication 1 pour la préparation d'un médicament destiné au traitement d'états pathologiques du SNC liés à un déséquilibre dans les concentrations physiologiques intraneuronales de la gastrine ou d'autres polypeptides bioactifs apparentés.

**9.** Procédé de préparation de dérivés d'acide glutamique ou d'acide aspartique de formule (I), dans laquelle n, $R_1$ et $R_2$ ont les significations données dans la revendication 1 et dans lesquels les substituants du centre chiral (marqué par un astérique) ont la conformation DL, caractérisé en ce qu'il comprend les étapes consistant à :

a) faire réagir un anhydride interne de formule (IV) :

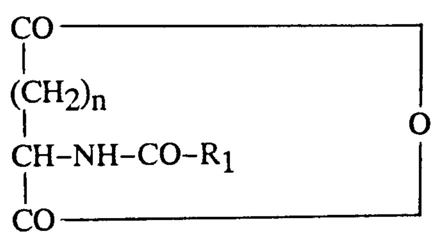

(IV)

dans laquelle n et $R_1$ ont les significations données ci-dessus, avec une amine primaire de formule $R_2$-$NH_2$, dans laquelle $R_2$ a la signification donnée ci-dessus, à un rapport molaire compris entre 1 et 5 à une température comprise entre -10 °C et 10 °C, et récupérer les composés (I) de la masse réactionnelle.

**10.** Procédé pour la préparation d'un dérivé d'acide glutamique ou aspartique de formule (1) dans laquelle n, $R_1$ et $R_2$ ont les significations données dans la revendication 1, et dans laquelle les substituants du centre chiral (marqué par un astérisque dans la formule (I)) ont la conformation (D), caractérisé en ce qu'il comprend les étapes conservant la stéréochimie consistant à :

a) faire réagir l'ester $\gamma$-benzylique d'acide N-carbobenzoxy-D-glutamique avec une amine de formule $H_2$-$NR_2$, dans laquelle $R_2$ a la signification donnée ci-dessus, par la méthode de l'anhydride mélangé, à une température comprise entre -15 °C et +15 °C et dans un solvant inerte anhydre, puis récupérer les composés (III) de la masse réactionnelle,

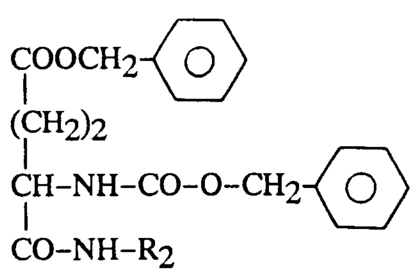

(III)

b) débenzyler et décarbobenzoxyler le composé de formule (III) dissous dans un solvant inerte, comme le méthanol, en le faisant réagir avec de l'hydrogène à température et à pression ambiantes

en présence d'une quantité catalytique efficace d'un catalyseur d'hydrogénation et récupérer les composés (II) de la masse réactionnelle,

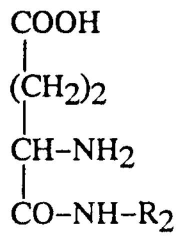

(II)

c) faire réagir les dérivés de formule (II) dans les conditions de Shotten-Baumann avec une quantité équimolaire d'un chlorure d'acyle de formule $R_1$-CO-Cl, dans laquelle $R_1$ a la signifieation donnée à la revendication 1, à une température comprise entre 0 et 15 °C et récupérer les dérivés de formule (I), ayant la conformation D, de la masse réactionnelle.

**Revendications pour l'Etat contractant suivant : ES**

**1.** Procédé de préparation d'un dérivé d'acide glutamique ou d'acide aspartique de formule (I) :

(*) 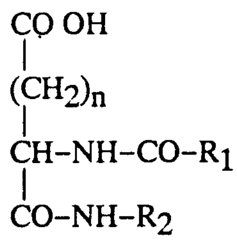

(I)

dans laquelle n vaut 1 ou 2, $R_1$ est un groupement phényle mono- ou disubstitué par des groupements chloro, méthyle ou nitro tels que l'atome d'hydrogène ou le groupement nitro peuvent être présents en position 2 et 6 du groupement phényle et, indépendamment, l'atome d'hydrogène, l'atome de chlore ou le groupement méthyle peut être présent aux positions 3 et 5, et dans laquelle $R_2$ représente une chaîne alkyle linéaire ou ramifiée ou un groupement cycloalkyle en $C_5$-$C_9$, et de ses sels pharmaceutiquement acceptables,
et dans laquelle les substituants du centre chiral (marque par un astérisque) ont la conformation DL, caractérisé en ce qu'il comprend les étapes consistant à :
a) faire réagir un anhydride interne de formule (IV) :

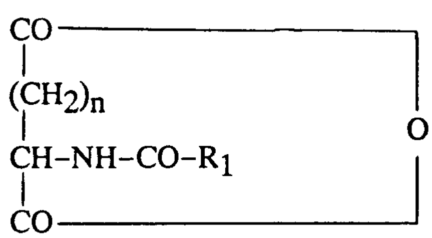

(IV)

dans laquelle n et $R_1$ ont les significations données ci-dessus, avec une amine primaire de formule $R_2$-$NH_2$, dans laquelle $R_2$ a la signification donnée ci-dessus, à un rapport molaire compris entre 1 et 5 à une température comprise entre -10 °C et 10 °C, et récupérer les composés (I) de la masse réactionnelle.

**2.** Procédé pour la préparation d'un dérivé d'acide glutamique ou aspartique de formule (I) dans laquelle n, $R_1$ et $R_2$ ont les significations données dans la revendication 1, et dans laquelle les substituants du centre chiral (marqué par un astérisque dans la formule (I)) ont la conformation (D), caractérisé en ce qu'il comprend les étapes conservant la stéréochimie consistant à :

a) faire réagir l'ester $\gamma$-benzylique d'acide N-carbobenzoxy-D-glutamique avec une amine de formule $H_2$-$NR_2$, dans laquelle $R_2$ a la signification donnée ci-dessus, par la méthode de l'anhydride mélangé, à une température entre -15 °C et +15 °C et dans un solvant inerte anhydre, puis récupérer les composés (III) de la masse réactionnelle,

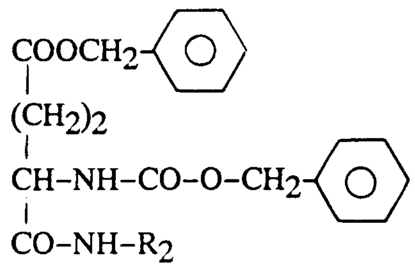

(III)

b) débenzyler et décarbobenzoxyler le composé de formule (III) dissous dans un solvant inerte, comme le méthanol, en le faisant réagir avec de l'hydrogène à température et à pression ambiantes en présence d'une quantité catalytique efficace d'un catalyseur d'hydrogénation et récupérer les composés (II) de la masse réactionnelle,

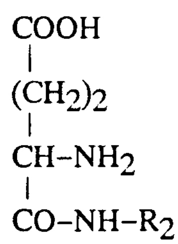

(II)

c) faire réagir les dérivés de formule (II) dans les conditions de Shotten-Baumann avec une quantité équimolaire d'un chlorure d'acyle de formule $R_1$-CO-Cl, dans laquelle $R_1$ a la signification donnée à la revendication 1, à une température comprise entre 0 et 15 °C et récupérer les dérivés de formule (I), ayant la conformation D, de la masse réactionnelle.

**3.** Procédé de préparation d'un composé selon la revendication 1 ou 2, dans lequel $R_1$ est choisi dans le groupe constitué des groupements 3-chlorophényle et 3,5-dichlorophényle, $R_2$ est choisi dans le groupe constitué des groupements 3,3-diméthylbutyle, 4,4-diméthylpentyle et 3,3-méthyléthylpentyle.

**4.** Procédé selon la revendication 1 pour la préparation d'un dérivé choisi parmi les suivants :
acide DL-4-[(3-chlorobenzoyl)amino]-5-(pentylamino)-5-oxopentanoïque ;
acide DL-4-[(3-chlorobenzoyl)amino]-5-(3-méthylbutylamino)-5-oxopentanoïque ;
acide DL-4-[(3-chlorobenzoyl)-amino]-5-(hexylamino)-5-oxopentanoïque ;
acide DL-4-[(3-chlorobenzoyl)-amino]-5-(3,3-diméthylbutylamino)-5-oxopentanoïque ;
acide DL-4-[(3-chlorobenzoyl)-amino]-5-(3-méthylpentylamino)-5-oxopentanoïque ;
acide DL-4-[(3-chlorobenzoyl)-amino]-5-(cyclohexylamino)-5-oxopentanoïque ;
acide DL-4-[(3-chlorobenzoyl)-amino]-5-(heptylamino)-5-oxopentanoïque ;
acide DL-4-[(3-chlorobenzoyl)-amino]-5-(4,4-diméthylpentyl)-5-oxopentanoïque ;
acide DL-4-[(3-chlorobenzoyl)-amino]-5-(3,3-diméthylpentylamino)-5-oxopentanoïque ;
acide DL-4-[(3-chlorobenzoyl)-amino]-5-(3-éthylpentyl)-5-oxopentanoïque ;
acide DL-4-[(3-chlorobenzoyl)-amino]-5-(cycloheptylamino)-5-oxopentanoïque ;
acide DL-4-[(3-chlorobenzoyl)-amino]-5-(4-méthylpentylamino)-5-oxopentanoïque ;
acide DL-4-[(3-chlorobenzoyl)-amino]-5-(3,3-méthyléthylpentylamino)-5-oxopentanoïque ;

acide DL-4-[(3-chlorobenzoyl)-amino]-5-(3,3-diéthylpentylamino)-5-oxopentanoïque ;
acide DL-4-[(3-chlorobenzoyl)-amino]-5-(2-(dicyclo[2.2.1]-heptyl)-éthylamino)-5-oxopentanoïque ;
acide DL-4-[(3-chlorobenzoyl)-amino]-5-(3,3-méthyléthyl-4-méthylpentylamino)-5-oxopentanoïque ;
acide DL-4-[(3,5-dichlorobenzoyl)-amino]-5-(3,3'-diméthylbutylamino)-5-oxopentanoïque ;
acide DL-3-[(3-chlorobenzoyl)-amino]-4-(3,3-méthyléthylpentylamino)-4-oxobutanoïque ;
acide DL-3-[(3-chlorobenzoyl)-amino]-4-(cycloheptylamino)-4-oxobutanoïque.

**5.** Procédé selon la revendication 2 pour la préparation d'un composé choisi parmi les suivants :
acide D-4-[(3-chlorobenzoyl)-amino]-5-(3,3-diméthylbutylamino)-5-oxopentanoïque ;
acide D-4-[(3-chlorobenzoyl)-amino]-5-(4,4-diméthylpentylamino)-5-oxopentanoïque.
acide D-4-[(3-chlorobenzoyl)-amino]-5-(3,3-méthyléthylpentylamino)-5-oxopentanoïque ;
acide D-4-[(3-chlorobenzoyl)-amino]-5-(cycloheptylamino)-5-oxopentanoïque ;
et de ses sels pharmaceutiquement acceptables.